# FASCICULE DE BREVET EUROPEEN

(11) **EP 0 768 297 B1**
(45) Date de publication et mention de la délivrance du brevet: **03.11.1999**
(21) Numéro de dépôt: 96401967.3
(22) Date de dépôt: 13.09.1996
(51) Int. Cl.: C07C 69/716, C07C 67/343

(54) **Dérivés des alkyls-3-oxo-alcanoates et leur procédé de préparation**
Derivate von Alkyl-3-oxo-alkanoaten und Verfahren zur ihrer Herstellung
Derivatives of alkyl-3-oxo-alcanoates and process for their preparation

(30) Priorité: 13.10.1995 FR 9512041
(43) Date de publication de la demande: 16.04.1997
(73) Titulaire: L'OREAL, 75008 Paris (FR)
(72) Inventeur: Philippe, Michel, 91320 Wissous (FR); Luppi, Bernadette, 93270 Sevran (FR); Semeria, Didier, 77181 Courtry (FR); Mahieu, Claude, 75017 Paris (FR)
(74) Mandataire: Tezier Herman, Béatrice

(56) Documents cités:
- EP-A- 0 646 572
- CHEMICAL ABSTRACTS, vol. 98, no. 1, 3 Janvier 1983 Columbus, Ohio, US; abstract no. 4451u, ZAV'YALOV S.I. ET AL.: "REACTIONS OF ESTERS OF ALPHA-(BROMOACYL)ACETIC ACID WITH SODIUM THIOACETATE" page 4452; colonne R; XP002005279 & IZV.AKAD.NAUK SSSR,SRE.KHIM., no. 8, 1982, USSR, page 1920 ZAV'YALOV S.I. ET AL.: "REACTIONS OF ESTERS OF ALPHA-(BROMOACYL)ACETIC ACID WITH SODIUM THIOACETATE"
- JOURNAL OF THE CHEMICAL SOCIETY, PERKIN TRANSACTIONS 1, no. 2, Février 1987, LETCHWORTH GB, pages 333-343, XP002005277 LESLIE CROMBIE ET AL.: "SYNTHESIS OF THE MAMMEA COUMARINS.PART 2.EXPERIMENTS IN THE MAMMEA E SERIES AND SYNTHESIS OF MAMMEA E/AC"
- JOURNAL OF ORGANIC CHEMISTRY, vol. 43, no. 10, 12 Mai 1978, EASTON US, pages 2087-2088, XP002005278 YUJI OIKAWA ET AL.: "Meldrum's Acid in Organic Chemistry.2.A General and Versatile Synthesis of beta-Keto Esters "
- J. Chem. Soc., 1951, 2453-2456

## Description

L'invention a pour objet de nouveaux précurseurs de céramides, leur procédé de préparation et leur utilisation pour la synthèse d'amino-acides, d'amino-alcools et de céramides.

Les céramides, à l'état naturel, sont les composants principaux des couches lipidiques de l'épiderme. Ils sont utilisés en cosmétique, sous forme naturelle ou synthétique, dans des compositions destinées, entre autre, à réduire le dessèchement de la peau ou à conférer à celle-ci une meilleure élasticité ou encore destinées au traitement du cheveu.

Les céramides naturels sont généralement obtenus par extraction de la peau de porc, du cerveau de boeuf, de l'oeuf, des cellules du sang, des plantes (JP 86/260008 ou JP87/120308).

Les inconvénients nombreux liés à ce type d'approvisionnement (fragilité, contamination, conservation, coût, etc.) ont fait que très tôt la voie de synthèse chimique a été explorée.

Toutefois, les voies de synthèse autorisant un développement industriel dans des limites de coût acceptables sont rares.

La préparation à l'échelle industrielle des amino-acides hydroxylés gras ainsi que des amino-alcools gras présente les mêmes difficultés.

On connait, par le document "J. Org. Chem., 43 (10), 1978, 2087-88", des composés de formule R1-CH₂-CO-CH₂-COOR2 avec R1 en C₂-C₁₀ et R2 en C₁-C₄.

On connait également, par le document "J. Chem. Soc., 1951,2453-56", la synthèse de mélanges racémiques de 2-amino-octadécane-1,3-diols; ce document cite notamment comme matière de départ le 3-oxo-octadécanoate de méthyle.

Aussi, c'est avec étonnement que la demanderesse a découvert de nouveaux composés, faciles à synthétiser et que l'on peut aisément utiliser pour la préparation de céramides, d'amino-acides hydroxylés gras, les acides 2-amino 3,4-diols alcanoïques, et d'amino-alcools gras, les 2-amino alcane 1,3,4-triols.

La présente invention a pour objet de nouveaux 3-oxo-alcanoates d'alcoyle répondant à la formule (I) : 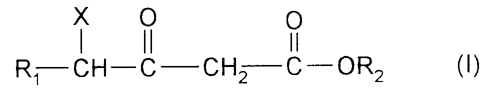 dans laquelle :
R₁ représente un radical alcoyle ou un radical alcényle linéaire ou ramifié, ou un radical aralcoyle, comportant de 6 à 28 atomes de carbone, R₁ pouvant être interrompu par des ponts éthers, R₁ portant éventuellement une ou plusieurs fonctions hydroxyles,
R₂ représente un radical alcoyle ou un radical alcényle linéaire ou ramifié, comprenant de 1 à 5 atomes de carbone,
X représente un groupe partant choisi parmi un atome de chlore, de brome ou d'iode, ou un groupement sulfonate

De façon préférentielle, les produits de formule (I) répondent à au moins l'une des conditions suivantes :
- R₁ représente un radical alcoyle ou un radical alcényle linéaire ou ramifié, comportant de 10 à 18 atomes de carbone, portant éventuellement une ou plusieurs fonctions hydroxyle,
- R₂ représente un radical alcoyle facilement hydrolysable, comme par exemple un radical méthyle ou éthyle,

De manière encore plus préférentielle, X désigne un atome de brome.

L'invention a également pour objet un procédé de préparation des produits (I) caractérisé en ce que l'on fait réagir un dérivé malonique avec un agent acylant.

Par dérivé malonique, on désigne un monoester ou un diester de l'acide malonique, comme par exemple l'ester d'isopropylidène de l'acide malonique appelé aussi acide de Meldrum, ou le sel de potassium du malonate d'éthyle. De façon préférentielle, on utilisera l'acide de Meldrum.

Par agent acylant, on désigne des composés répondant à la formule (II) : 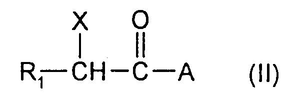 dans laquelle R₁ et X ont la même signification que ci-dessus, et A est choisi parmi les halogènes, les groupements 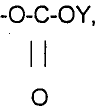 avec Y radical alcoyle en C₂ à C₈, les groupements alcoxy -OZ, avec Z désignant un radical alcoyle en C₁-C₈ ou un radical paranitrophényle ou un radical succinimidyle ou un radical dicyclohexylcarbodiimidyle, A peut également désigner un radical : 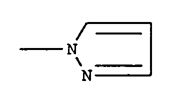 ou un radical

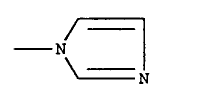

De façon préférentielle, X représente un atome de brome et A un atome de chlore.

Les produits répondant à la formule (II) décrits ci-dessus sont préparés par activation de l'acide correspondant, soit à partir d'un produit de formule (II) avec A = OH, par des moyens d'activation de l'acide bien connus de l'homme du métier. Les produits répondant à la formule (II) avec A = OH sont des produits disponibles commercialement.

La réaction du procédé de préparation des produits de formule (I) est conduite de préférence en milieu anhydre.
Elle est préférentiellement réalisée dans un solvant approprié, comme par exemple le tétrahydrofurane, le dichlorométhane, la pyridine, le tertiobutylméthyl éther.

L'invention a également pour objet l'utilisation des composés (I) pour la préparation de céramides, d'amino acides hydroxylés gras et d'amino alcools gras.

Par exemple, on peut substituer à X un groupement 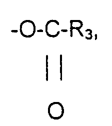 dans lequel R₃ représente un radical alcoyle ou un radical alcényle, ou un radical aryle, linéaire ou ramifié comprenant 1 à 6 atomes de carbone, puis on procédera à une oximation du groupement méthylène pour obtenir un composé de formule (III) 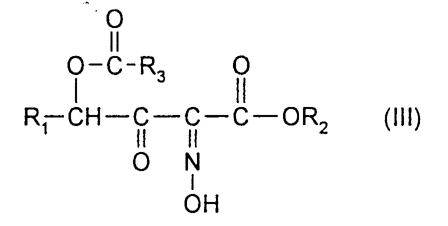 dans lequel R₁, R₂ et R₃ ont la même définition que ci-dessus.

A partir des composés de formule (III) et par des méthodes successives de réduction bien connues de l'homme du métier, on peut obtenir un 2-amino-1,3,4-triol, ou un de ses sels d'amine, répondant à la formule (IV).

R₁-CHOH-CHOH-CHNH₂-CH₂-OH (IV)

Ainsi, on pourra par exemple utiliser les méthodes de réduction employées par Posternik (Chem. Phys. lipids 1971, 7, 135-143), lsida (J. Org. Chem. 1969, 34, 3539-3544) et Jäger (Angew Chem. Int. Ed. Engl. 1981, 20, 601-605) pour la synthèse d'amino alcane triols. Par exemple, on pourra traiter (III) par de l'hydrogène sous pression en présence d'un catalyseur ou par de l'hydrogène naissant, généré par du zinc en présence d'un acide, comme par exemple de l'acide acétique.

De façon analogue, on pourra préparer des amino-acides gras hydroxylés en traitant les composés de formule (III) avec des réducteurs choisis parmi : l'hydrogène sous pression en présence d'un catalyseur et l'hydrogène naissant, généré par du zinc en présence d'un acide, comme par exemple de l'acide acétique.

A partir des composés de formule (III), on peut également synthétiser de façon directe des céramides, par un procédé en deux étapes consistant à convertir l'alkyl-2-hydroximino-3-oxo-4-alcanoyloxy-alcanoate en l'alkyl-2-alcanoylamido-3-oxo-4-alcanoyloxy-alcanoate par réduction et acylation de l'oxime, puis à réduire les fonctions ester et cétone en alcool, par exemple par traitement au borohydrure de sodium. On pourra, par exemple, se référer au document EP-A-646572 qui donne des exemples de telles synthèses. On pourra également se référer à Soukup (Helv. Chim. Acta, 70, 232-236, 1987) pour les réactions d'oximation, réduction de l'oxime et acylation de l'amine permettant de préparer un alkyl-2-alcanoylamido-3-oxo-4-alcanoyloxy-alcanoate à partir des produits selon l'invention.

Le composé de formule (IV) obtenu selon l'invention peut être utilisé dans les procédés de synthèse pouvant conduire à des céramides comme ceux décrits par Shapiro, D. (Chemistry of sphingolipids, Hermann, Paris, 1969,p 26-34).

Les exemples qui suivent illustrent la présente invention sans toutefois en limiter la portée.

### Exemples :

### Exemple 1 : Synthèse du 4-bromo-3-oxo-octadécanoate de méthyle

Dans un ballon contenant du dichloro-1,2 éthane, placé à 0°C, sous argon et sous agitation, on mélange 1,8moles d'acide de Meldrum avec 3,96moles de pyridine. On introduit ensuite lentement 1,98moles du chlorure de l'acide 2-bromo-hexadécanoïque et on laisse agiter pendant deux heures à 0°C. La solution est ensuite lavée trois fois par de l'eau, séchée, le solvant évaporé et le résidu repris dans du méthanol. La solution dans le méthanol est portée au reflux pendant deux heures puis le solvant est évaporé sous vide. Le 4-bromo-3-oxo-octadécanoate de méthyle est alors purifié sur colonne de silice. On récupère ainsi 494g de 4-bromo-3-oxo-octadécanoate de méthyle, sous la forme d'un mélange des formes cétonique (c) 40% et énolique (e) 60%.

### Spectres RMN :

Spectre H¹ ( CDCl3):
δ = 0,87, t, CH3 (c+e); δ = 1,25-1,6, m, -(CH2)₁₂- (c+e); δ = 1,8-2,12, m, (C13 H27)-CH2-CHBr (c+e); δ = 3,63-3,83, ab, -CH2-CO2CH3 (c); δ = 3,74-3,76, 2s, OCH3 (c+e); δ = 4,21, m, CHBr (e); δ = 4,43, dd, CHBr (c); δ = 5,23-5,29, 2s, =CH-CO2CH3 (e); δ = 11,96-11,97, s large, OH (e).

Spectre C¹³ (CDCl3):
δ = 14,06, CH3 ; δ = 22,66, CH3-CH2 ; δ = 27,14, C12H25-CH2- (c); δ = 27,53, C12H24-CH2 (e) ; δ = 28,8-29,66, -(CH2)₉- ; δ =31,9, C2H5-CH2- ; δ = 32,97, -CH2-CHBr (c) ; δ = 35,5, -CH2-CHBr (e) ; δ = 45,19, -CH2CO2CH3 (c) ; δ = 49,91, -CHBr (e) ; δ = 51,53, OCH3 (e) ; δ = 52,43, OCH3 (c) ; δ = 53,3, -CHBr (c) ; δ = 89,83, OCH3 (e) ; δ = 167,34, -CO2CH3 (c) ; δ = 172,65-173,69, =COH et -CO2CH3 (e) ; δ = 196,01, -CO-(c) ;

## Revendications

1. Dérivés 3-oxo-alcanoates d'alcoyle répondant à la formule (I) : 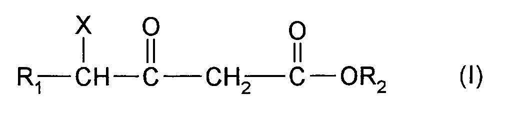 dans laquelle :
- R₁ représente un radical alcoyle ou un radical alcényle linéaire ou ramifié, ou un radical aralcoyle, comportant de 6 à 28 atomes de carbone, R₁ pouvant être interrompu par des ponts éthers, R₁ portant éventuellement une ou plusieurs fonctions hydroxyles,
- R₂ représente un radical alcoyle ou un radical alcényle linéaire ou ramifié, comprenant 1 à 5 atomes de carbone,
- X représente un groupe partant choisi parmi un atome de chlore, de brome ou d'iode, ou un groupement sulfonate.

2. Produit selon la revendication 1, caractérisé en ce que R₁ représente un radical alcoyle ou un radical alcényle linéaire ou ramifié, comportant de 10 à 18 atomes de carbone, portant éventuellement une ou plusieurs fonctions hydroxyle.

3. Produit selon l'une quelconque des revendications 1 et 2, caractérisé en ce que R₂ représente un radical méthyle ou éthyle.

4. Produit selon l'une quelconque des revendications 1 à 3, caractérisé en ce que X désigne un atome de brome.

5. Procédé de préparation des produits selon l'une quelconque des revendications 1 à 4, caractérisé en ce que l'on fait réagir un monoester ou un diester de l'acide malonique, avec un agent acylant répondant à la formule (II) : 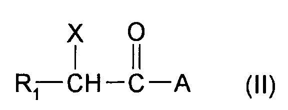 dans laquelle R₁ et X ont la même signification que dans les revendications précédentes, et A est choisi parmi les radicaux : 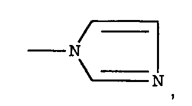 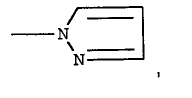 les halogènes, les groupements -O-C(O)-OY, avec Y radical alcoyle en C₂ à C₈ et les groupements alcoxy -OZ tels que Z est choisi parmi les radicaux alcoyle en C₁-C₈, le radical paranitrophényle, le radical succinimidyle et le radical dicyclohexylcarbodiimidyle.

6. Procédé selon la revendication 5, caractérisé en ce que le monoester ou le diester de l'acide malonique est l'ester d'isopropylidène de l'acide malonique ou le sel de potassium du malonate d'éthyle.

7. Procédé selon l'une des revendications 5 à 6, caractérisé en ce que le monoester ou le diester de l'acide malonique est l'ester d'isopropylidène de l'acide malonique.

8. Procédé selon l'une des revendications 5 à 7, caractérisé en ce que X représente un atome de brome et A un atome de chlore.

9. Procédé selon l'une des revendications 5 à 8, caractérisé en ce que la réaction est conduite en milieu anhydre.

10. Procédé selon l'une quelconque des revendications 5 à 9, caractérisé en ce que la réaction est réalisée dans un solvant choisi parmi le tétrahydrofurane, le dichlorométhane, la pyridine, le tertiobutylméthyl éther.

11. Utilisation de dérivés 3-oxo-alcanoates d'alcoyle répondant à la formule (I) : 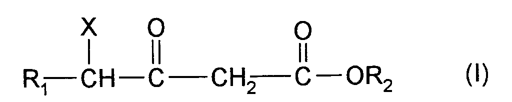 dans laquelle :
- R₁ représente un radical alcoyle ou un radical alcényle linéaire ou ramifié, ou un radical aralcoyle, comportant de 6 à 28 atomes de carbone, R₁ pouvant être interrompu par des ponts éthers, R₁ portant éventuellement une ou plusieurs fonctions hydroxyles,
- R₂ représente un radical alcoyle ou un radical alcényle linéaire ou ramifié, comprenant 1 à 5 atomes de carbone,
- X représente un groupe partant, choisi parmi un atome de chlore, de brome ou d'iode, ou un groupement sulfonate,
pour la préparation de 2-amino-alcanes 1,3,4-triols, ou d'un de leurs sels d'amine, de formule R₁-CHOH-CHOH-CHNH₂-CH₂-OH ; et/ou
pour la préparation d'acides 2-amino 3,4-diols alcanoïques.

12. Utilisation de dérivés 3-oxo-alcanoates d'alcoyle répondant à la formule (I) : 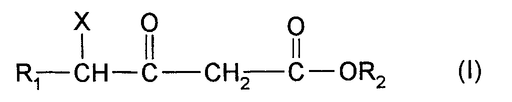 dans laquelle :
- R₁ représente un radical alcoyle ou un radical alcényle linéaire ou ramifié, ou un radical aralcoyle, comportant de 6 à 28 atomes de carbone, R₁ pouvant être interrompu par des ponts éthers, R₁ portant éventuellement une ou plusieurs fonctions hydroxyles,
- R₂ représente un radical alcoyle ou un radical alcényle linéaire ou ramifié, comprenant 1 à 5 atomes de carbone,
- X représente un groupe partant, choisi parmi un atome de chlore, de brome ou d'iode, ou un groupement sulfonate,
pour la préparation de composé de formule (III) : 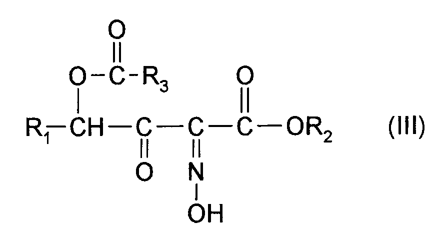 dans laquelle R₃ représente un radical alcoyle ou un radical alcényle, ou un radical aryle, linéaire ou ramifié comprenant 1 à 6 atomes de carbone.

13. Utilisation selon la revendication 12,
- pour la préparation de 2-amino-alcanes 1,3,4-triols, ou d'un de leurs sels d'amine, de formule : R₁-CHOH-CHOH-CHNH₂-CH₂-OH ;
- pour la préparation d'acides 2-amino 3,4-diols alcanoïques; et/ou
- pour la préparation d'alkyl-2-alcanoylamido-3-oxo-4-alcanoyloxy-alcanoate, à partir de composé de formule (III).

14. Utilisation selon l'une des revendications 11 à 13, pour la préparation de céramides.

## Claims

1. Alkyl 3-oxoalkanoate derivatives corresponding to formula (I): 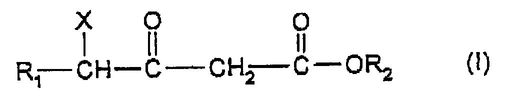 in which:
R₁ represents an alkyl radical or an alkenyl radical which is linear or branched, or an aralkyl radical, containing from 6 to 28 carbon atoms, it being possible for R₁ to be interrupted by ether bridges, R₁ optionally bearing one or more hydroxyl functions,
R₂ represents an alkyl radical or an alkenyl radical which is linear or branched, comprising from 1 to 5 carbon atoms,
X represents a leaving group chosen from a chlorine, bromine or iodine atom and a sulphonate group.

2. Product according to Claim 1, characterized in that R₁ represents an alkyl radical or an alkenyl radical, which is linear or branched, containing from 10 to 18 carbon atoms, optionally bearing one or more hydroxyl functions.

3. Product according to either of Claims 1 and 2, characterized in that R₂ represents a methyl or ethyl radical.

4. Product according to any one of Claims 1 to 3, characterized in that X denotes a bromine atom.

5. Process for the preparation of the products according to any one of Claims 1 to 4, characterized in that a monoester or a diester of malonic acid is reacted with an acylating agent corresponding to formula (II): 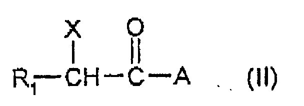 in which R₁ and X have the same meanings as in the preceding claims and A is chosen from the radicals: 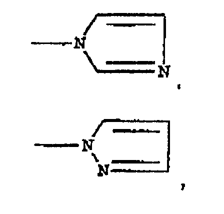 halogens, groups -O-C(O)-OY, where Y is a C₂ to C₈ alkyl radical and alkoxy groups -OZ such that Z is chosen from C₁-C₈ alkyl radicals, the para-nitrophenyl radical, the succinimidyl radical and the dicyclohexyl-carbodiimidyl radical.

6. Process according to Claim 5, characterized in that the monoester or diester of malonic acid is the isopropylidene ester of malonic acid or the potassium salt of ethyl malonate.

7. Process according to either of Claims 5 or 6, characterized in that the monoester or diester of malonic acid is the isopropylidene ester of malonic acid.

8. Process according to one of Claims 5 to 7, characterized in that X represents a bromine atom and A a chlorine atom.

9. Process according to one of Claims 5 to 8, characterized in that the reaction is carried out in an anhydrous medium.

10. Process according to any one of Claims 5 to 9, characterized in that the reaction is performed in a solvent chosen from tetrahydrofuran, dichloromethane, pyridine and tert-butyl methyl ether.

11. Use of alkyl 3-oxoalkanoate derivatives corresponding to formula (I): 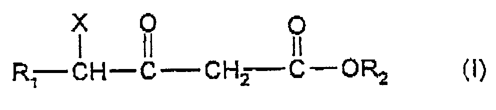 in which:
- R₁ represents a linear or branched alkyl radical or alkenyl radical, or an aralkyl radical, comprising from 6 to 28 carbon atoms, it being possible for R₁ to be interrupted with ether bridges, R₁ optionally bearing one or more hydroxyl functions,
- R₂ represents an alkyl radical or a linear or branched alkenyl radical, comprising 1 to 5 carbon atoms,
- X represents a leaving group chosen from a chlorine, bromine or iodine atom and a sulphonate group,
for the preparation of 2-aminoalkane-1,3,4-triols, or one of the amine salts thereof, of formula R₁-CHOH-CHOH-CHNH₂-CH₂-OH; and/or
for the preparation of 2-amino-3,4-diolalkanoic acids.

12. Use of alkyl 3-oxoalkanoate derivatives corresponding to formula (I): 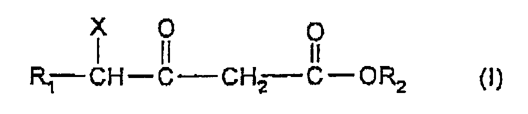 in which:
- R₁ represents a linear or branched alkyl radical or alkenyl radical, or an aralkyl radical, comprising from 6 to 28 carbon atoms, it being possible for R₁ to be interrupted with ether bridges, R₁ optionally bearing one or more hydroxyl functions,
- R₂ represents a linear or branched alkyl radical or alkenyl radical comprising 1 to 5 carbon atoms,
- X represents a leaving group chosen from a chlorine, bromine or iodine atom and a sulphonate group, for the preparation of a compound of formula (III): 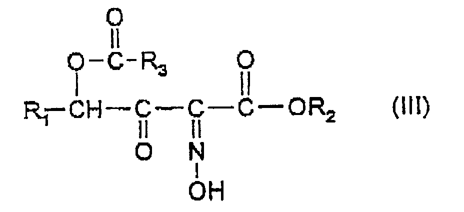 in which R₃ represents a linear or branched alkyl radical or alkenyl radical, or an aryl radical, comprising 1 to 6 carbon atoms.

13. Use according to Claim 12,
- for the preparation of 2-aminoalkane-1,3,4-triols, or one of the amine salts thereof, of formula:
R₁-CHOH-CHOH-CHNH₂-CH₂-OH;
- for the preparation of 2-amino-3,4-diolalkanoic acids; and/or
- for the preparation of alkyl 2-alkanoylamido-3-oxo-4-alkanoyloxyalkanoate,
from a compound of formula (III).

14. Use according to one of Claims 11 to 13 for the preparation of ceramides.

## Patentansprüche

1. Alkyl-3-oxo-alkanoatderivate der Formel (I): 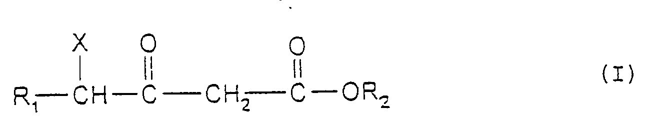 worin bedeuten:
- R₁ eine geradkettige oder verzweigte Alkyl- oder Alkenylgruppe oder eine Aralkylgruppe mit 6 bis 28 Kohlenstoffatomen, wobei R₁ durch Etherbindungen unterbrochen sein kann und gegebenenfalls eine oder mehrere Hydroxygruppen enthalten kann,
- R₂ eine geradkettige oder verzweigte Alkyl- oder Alkenylgruppe mit 1 bis 5 Kohlenstoffatomen,
- X eine austretende Gruppe, die unter Chlor, Brom oder Iod ausgewählt ist, oder eine Sulfonatgruppe.

2. Produkt nach Anspruch 1, dadurch gekennzeichnet, daß R₁ eine geradkettige oder verzweigte Alkyl- oder Alkenylgruppe mit 10 bis 18 Kohlenstoffatomen bedeutet, die gegebenenfalls eine oder mehrere Hydroxygruppen aufweist.

3. Produkt nach einem der Ansprüche 1 und 2, dadurch gekennzeichnet, daß R₂ Methyl oder Ethyl bedeutet.

4. Produkt nach einem der Ansprüche 1 bis 3, dadurch gekennzeichnet, daß X ein Bromatom bedeutet.

5. Verfahren zur Herstellung von Produkten nach einem der Ansprüche 1 bis 4, dadurch gekennzeichnet, daß ein Monoester oder ein Diester von Malonsäure mit einem Acylierungsmittel der Formel (II) 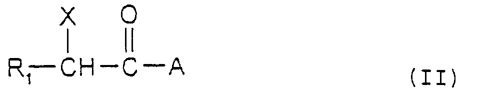 umgesetzt wird, worin R₁ und X die in den vorhergehenden Ansprüchen angegebenen Bedeutungen aufweisen und A unter den folgenden Gruppen ausgewählt ist: 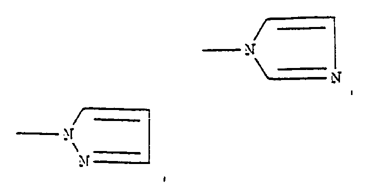 den Halogenen, den Gruppen -O-C(O)-OY, worin Y eine Alkylgruppe mit 2 bis 8 Kohlenstoffatomen bedeutet, und den Alkoxygruppen -OZ, worin Z unter C₁₋₈-Alkylgruppen, p-Nitrophenyl, Succinimidyl und Dicyclohexylcarbodiimidyl ausgewählt ist.

6. Verfahren nach Anspruch 5, dadurch gekennzeichnet, daß der Monoester oder der Diester von Malonsäure der Malonsäureisopropylidenester oder das Kaliumsalz von Ethylmalonat ist.

7. Verfahren nach einem der Ansprüche 5 bis 6, dadurch gekennzeichnet, daß der Monoester oder der Diester von Malonsäure der Malonsäureisopropylidenester ist.

8. Verfahren nach einem der Ansprüche 5 bis 7, dadurch gekennzeichnet, daß X ein Bromatom und A ein Chloratom bedeutet.

9. Verfahren nach einem der Ansprüche 5 bis 8, dadurch gekennzeichnet, daß die Umsetzung in einem wasserfreien Medium durchgeführt wird.

10. Verfahren nach einem der Ansprüche 5 bis 9, dadurch gekennzeichnet, daß die Umsetzung in einem Lösungsmittel durchgeführt wird, das unter Tetrahydrofuran, Dichlormethan, Pyridin und tert.-Butylmethylether ausgewählt ist.

11. Verwendung von Alkyl-3-oxo-alkanoatderlvaten der Formel (I) 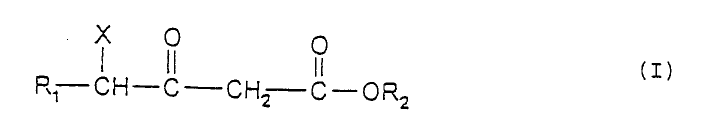 worin bedeuten:
- R₁ eine geradkettige oder verzweigte Alkyl- oder Alkenylgruppe oder eine Aralkylgruppe mit 6 bis 28 Kohlenstoffatomen, wobei R₁ durch Etherbindungen unterbrochen sein kann und gegebenenfalls eine oder mehrere Hydroxygruppen enthalten kann,
- R₂ eine geradkettige oder verzweigte Alkyl- oder Alkenylgruppe mit 1 bis 5 Kohlenstoffatomen,
- X eine austretende Gruppe, die unter Chlor, Brom oder Iod ausgewählt ist, oder eine Sulfonatgruppe,
zur Herstellung von 2-Aminoalkan-1,3,4-triolen oder einem ihrer Aminsalze der Formel R₁-CHOH-CHOH-CHNH₂-CH₂-OH und/oder zur Herstellung von 2-Amino-3,4-diolalkansäuren.

12. Verwendung von Alkyl-3-oxo-alkanoatderivaten der Formel (I) 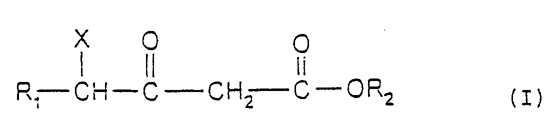 worin bedeuten:
- R₁ eine geradkettige oder verzweigte Alkyl- oder Alkenylgruppe oder eine Aralkylgruppe mit 6 bis 28 Kohlenstoffatomen, wobei R₁ durch Etherbindungen unterbrochen sein kann und gegebenenfalls eine oder mehrere Hydroxygruppen enthalten kann,
- R₂ eine geradkettige oder verzweigte Alkyl- oder Alkenylgruppe mit 1 bis 5 Kohlenstoffatomen,
- X eine austretende Gruppe, die unter Chlor, Brom oder Iod ausgewählt ist, oder eine Sulfonatgruppe zur Herstellung einer Verbindung der Formel (III) 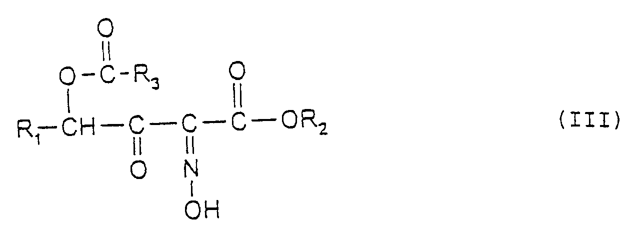 worin R₃ eine geradkettige oder verzweigte Alkylgruppe, Alkenylgruppe oder Arylgruppe mit 1 bis 6 Kohlenstoffatomen bedeutet.

13. Verwendung nach Anspruch 12,
- zur Herstellung von 2-Aminoalkan-1,3,4-triolen oder einem ihrer Aminsalze der Formel R₁-CHOH-CHOH-CHNH₂-CH₂-OH;
- zur Herstellung von 2-Amino-3,4-diolalkansäuren und/oder
- zur Herstellung von Alkyl-2-alkanoylamino-3-oxo-4-alkanoyloxyalkanoaten
aus der Verbindung der Formel (III).

14. Verwendung nach einem der Ansprüche 11 bis 13 zur Herstellung von Ceramiden.
